# EUROPÄISCHE PATENTSCHRIFT

(11) **EP 0 576 974 B1**
(45) Veröffentlichungstag und Bekanntmachung des Hinweises auf die Patenterteilung: **05.02.2003**
(21) Anmeldenummer: 93109979.0
(22) Anmeldetag: 23.06.1993
(51) Int. Cl.: C07D 453/02, A61K 7/42, A61K 31/435

(54) **Benzyliden-chinuclidinone als Sonnenschutzmittel**
Benzyliden-quinuclidinones as sunscreen agents
Benzylidène-quinuclidinones comme protecteurs solaires

(30) Priorität: 02.07.1992 DE 4221740
(43) Veröffentlichungstag der Anmeldung: 05.01.1994
(73) Patentinhaber: MERCK PATENT GmbH, 64271 Darmstadt (DE)
(72) Erfinder: Stein, Ingeborg, Dr., D-6106 Erzhausen (DE); Martin, Roland, Dr., D-6940 Weinheim (DE); Casutt, Michael, Dr., D-6148 Heppenheim (DE); Heywang, Ulrich, Dr., D-64271 Darmstadt (DE); Böttcher, Henning, Dr., D-64271 Darmstadt (DE); Hölzemann, Gunther, Dr., D-6104 Seeheim-Jugenheim (DE)

(56) Entgegenhaltungen:
- EP-A- 0 390 681
- DE-A- 3 833 706
- FR-A- 2 658 075
- US-A- 5 175 340
- CHEMICAL ABSTRACTS, vol. 108, 1988, Columbus, Ohio, US; abstract no. 68304f, P.H.DOUKAS ET AL. 'Suppression of acetylcholine by novel quinuclidine derivatives: comparison ...'
- CHEMICAL ABSTRACTS, vol. 106, 1987, Columbus, Ohio, US; abstract no. 43843b, YANINA,A.D. ET AL. 'Synthesis and pharmacological properties of 2-benzylquinuclidine analogs of dopamine.'
- CHEMICAL ABSTRACTS, vol. 105, 1986, Columbus, Ohio, US; abstract no. 78870q, TURCHIN,A.D. ET AL. 'Synthesis, structure and properties of pyrazolo(4,3-b)quinuclidine derivatives.'
- JOURNAL OF ORGANIC CHEMISTRY. Bd. 39, Nr. 24, 1974, EASTON US Seiten 3511 - 3516 E.J. WARAWA ET AL. 'Quinuclidine chemistry: I. Configuration and chemistry of 2-substituted benzylidene-3-quinuclidinones'

## Beschreibung

Die Erfindung betrifft eine kosmetische Zubereitung enthaltend mindestens ein Benzyliden-chinuclidinon-Derivat der Formel I 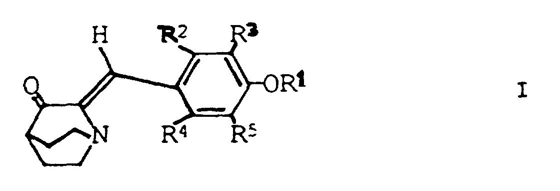 worin
- R¹: A,
- R², R³, R⁴ und R⁵: jeweils unabhängig voneinander H, A, OA oder OH
und
- A: einen geradkettigen oder verzweigten Alkylrest mit 1 bis 10 C-Atomen

bedeuten, ihre Salze sowie Verfahren zu ihrer Herstellung und ihre Verwendung in kosmetischen und/oder pharmazeutischen Zubereitungen, insbesondere zum Schutz vor Sonneneinstrahlung, bzw. UV-Strahlen und zur Prävention und/oder Behandlung von Entzündungen und Allergien der Haut oder bestimmten Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Effekte: sie bewirken beispielsweise, daß die Haut ihre Elastizität verliert und sich Falten bilden und führen somit zu einer frühzeitigen Alterung. Manchmal kann man auch Dermatosen beobachten. Im extremen Fall kann es zum Auftreten von Hautkrebs kommen.

Es ist bekannt, daß die in kosmetischen Präparaten enthaltenen Komponenten nicht immer genügend lichtstabil sind und sich unter der Einwirkung von Lichtstrahlen zersetzen und somit ihre Wirkung verlieren.

Bekanntlich wird der gefährlichste Teil der Sonnenstrahlen von den ultravioletten Strahlen mit einer Wellenlänge von weniger als 400 nm gebildet. Bekannt ist ferner, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können.

UV-B-Strahlen mit einer Wellenlänge zwischen 280 und 320 nm spielen insbesondere bei der Bildung von Sonnenerythemen eine besondere Rolle. UV-A-Strahlen mit einer Wellenlänge zwischen 320 nm und 400 nm sorgen insbesondere für die Bräunung der Haut, aber auch für deren Alterung. Ferner begünstigen sie die Auslösung von erythematösen Hautreaktionen und rufen in manchen Fällen phototoxische oder photoallergische Hautveränderungen hervor.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet, denn Verbindungen dieser Art, wie z.B. die Dibenzoylmethane Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut begünstigen kann. Die ebenfalls als UVA-Filter verwendeten Benzophenone sind in den in der Kosmetik verwandten Ölen nur begrenzt löslich und weisen eine relativ geringe Absorption auf. Dagegen sind nur wenige wasserlösliche UVA-Filter derzeit bekannt, deren UV-Absorption jedoch gering ist.

Aus der DE-OS 38 33 706 sind Benzylidenkampfer-Derivate bekannt. Diese weisen jedoch mindestens eine tertiäre Alkylgruppe auf. Derartige Verbindungen können zwar auch als UV-Filter in Sonnenschutzmitteln verwendet werden, eignen sich jedoch aufgrund der phenolischen Hydroxylgruppe eher als Antioxidantien. Aufgrund ihrer tertiären Alkylgruppe sind diese Verbindungen in herkömmlichen kosmetischen Trägern, insbesondere in wäßrigen Suspensionen, nur begrenzt löslich, so daß sie in Sonnenschutzmitteln immer zusammen mit anderen UV-Filtern eingesetzt werden müssen.

Der Erfindung lag die Aufgabe zugrunde, neue Verbindungen aufzufinden, die zur Herstellung von kosmetischen und/oder pharmazeutischen Zubereitungen verwendet werden können und die genannten Nachteile nicht aufweisen.

Es wurde gefunden, daß die Verbindungen der Formel I und ihre physiologisch unbedenklichen Säureadditionssalze wertvolle kosmetische und pharmazeutische Eigenschaften besitzen. So sind die Verbindungen der Formel I Substanzen mit hervorragenden UVA-Filter-Eigenschaften. Ihre Löslichkeit in den in der Kosmetik verwandten Ölen ist sehr gut, so daß Einsatzkonzentrationen auch in komplizierten Formulierungen bis mindestens 10 % der Zubereitung möglich sind.

Verbindungen der Formel I mit längerkettigen Alkoxygruppen, wie z.B. der 2-Ethylhexyloxy-Gruppe sind zum Teil in jedem Verhältnis mit gebräuchlichen kosmetischen Lösungsmitteln, wie Miglyol oder Paraffin mischbar.

Die Extinktion der Verbindungen zeigt darüber hinaus im UVB-Bereich ein Minimum; dies ist jedoch kein Nachteil, da man problemlos einen UVB-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Trägern auf Fettbasis einen kontinuierlichen Film bilden; sie können auf diese Weise auf die Haut aufgetragen werden, um einen wirksamen Schutzfilm zu bilden.

Gegenstand der Erfindung sind kosmetische Zubereitungen enthaltend mindestens einer Verbindung der obengenannten Formel I.

In dieser Formel steht R¹ für A. A bedeutet einen geradkettigen oder verzweigten Alkylrest mit 1-10 C-Atomen. Insbesondere bedeutet A Methyl, Ethyl, Propyl, Isopropyl, Butyl, Isobutyl, sek.-Butyl, tert.-Butyl, Pentyl, Hexyl, 2-,3-,4-,5-Methyl-hexyl, 2-,3-,4-,5-Ethylhexyl oder Heptyl.

R², R³, R⁴ und R⁵ bedeuten jeweils unabhängig voneinander ein Wasserstoffatom, A, OH oder OA, wobei A vorzugsweise einem der zuvor bevorzugt genannten Alkylreste entspricht.

Dementsprechend sind Gegenstand der Erfindung insbesondere diejenigen Verbindungen der Formel II 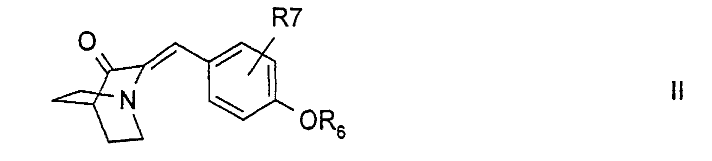 worin
- R6: 2-Ethylhexyl,
- R⁷: H oder OA und
- A: C₁₋₄ Alkyl,
bedeuten, sowie ihre Salze.

Zu den besonders bevorzugten Verbindungen der Formel II gehören:
2-(4-(2-Ethylhexyloxy)-benzyliden)-chinuclidin-3-on;
2-(4-(2-Ethylhexyloxy)-3-methoxy-benzyliden)-chinuclidin-3-on;

Gegenstand der Erfindung ist ferner ein Verfahren zur Herstellung von Benzyliden-chinuclidinon-Derivaten der Formel II sowie von deren Salzen, dadurch gekennzeichnet, daß man 3-Chinuclidinon mit einer Verbindung der Formel III 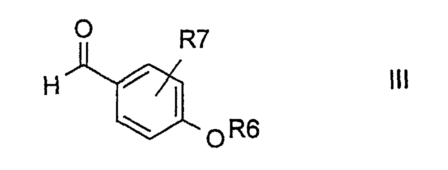 worin die Reste R¹ und R² die in Anspruch 8 angegebene Bedeutung haben,
umsetzt,
oder daß man eine sonst der Formel II entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man eine erhaltene Base der Formel II durch Behandlung mit einer Säure in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Ein Verfahren zur Herstellung von Benzyliden-chinuclidinon-Derivaten der Formel I sowie von deren Salzen, ist beispielsweise dadurch gekennzeichnet, daß man 3-Chinuclidinon mit einer Verbindung der Formel IV 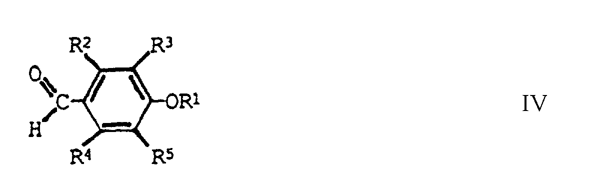 worin die Reste R¹, R², R³, R⁴ und R⁵ die angegebenen Bedeutungen haben,
umsetzt,
oder daß man eine sonst der Formel I entsprechende Verbindung, die jedoch anstelle eines oder mehrerer Wasserstoffatome eine oder mehrere solvolysierbare Gruppe (n) enthält, mit einem solvolysierenden Mittel behandelt,
oder daß man eine Verbindung, die sonst der Formel I nach Anspruch 1 entspricht, aber anstelle eines oder mehrerer H-Atome eine abspaltbare Schutzgruppe enthält, durch Abspaltung dieser Schutzgruppe in eine Verbindung der Formel I überführt,
oder daß man in einer Verbindung der Formel I gemäß Anspruch 1 einen oder mehrerer der Reste R², R³, R⁴ und/oder R⁵ in einen oder mehrere andere Reste R², R³, R⁴ und/oder R⁵ umwandelt
und/oder daß man eine erhaltene Base der Formel I durch Behandlung mit einer Säure in eines ihrer Salze umwandelt.

Die Ausgangsstoffe für das Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel IV ist R¹ vorzugsweise Ethyl, Propyl, Isopropyl, Butyl, tert. Butyl, sec. Butyl, Isobutyl oder besonders bevorzugt Methyl oder 2-Ethyl-hexyl.

Die Reste R², R³, R⁴ und R⁵ sind vorzugsweise H oder OA, wie z.B. Methoxy, Ethoxy, Propyloxy oder aber auch 2-Ethyl-hexyloxy. In besonders bevorzugten Verbindungen der Formel II bedeuten ein oder zwei der Reste R², R³, R⁴ und/oder R⁵ OA, während die übrigen Wasserstoffatomen entsprechen.

Die Verbindungen der Formel IV sind zum größten Teil bekannt; die nicht bekannten Verbindungen der Formel II können leicht in Analogie zu den bekannten hergestellt werden.

Die Aldehyde der Formel IV können beispielsweise durch Oxidation der entsprechenden Alkohole oder durch Reduktion der entsprechenden Carbonsäurederivate nach an sich bekannten Methoden hergestellt werden. Weitere geeignete Verfahren zur Synthese der aromatischen Aldehyde der Formel IV sind beispielsweise die Reimer-Tiemann-, Vilsmeier-Haack- oder Gattermann-Koch-Synthese, aber auch andere hier nicht näher erwähnte Verfahren können ebenfalls zur Darstellung herangezogen werden.

Die Umsetzung der Verbindungen IV mit 3-Chinuclidinon verläuft nach Methoden, wie sie für die Kondensation von Aldehyden und Ketonen aus der Literatur bekannt sind (vgl. hierzu Houben-Weyl, Methoden der Organ. Chemie Vol. 7/26, Georg-Thieme-Verlag Stuttgart (1976); Modern Synthetic React. 2nd ed., 629-682 (1972)).

Man kann z.B. ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner können Verbindungen, die sonst der Formel I entsprechen, aber anstelle eines oder mehrerer H-Atome eine oder mehrere solvolysierbare Gruppe(n) enthalten, zu Verbindungen der Formel I solvolysiert, insbesondere hydrolysiert werden.

Die Ausgangsstoffe für die Solvolyse sind beispielsweise erhältlich durch Reaktion von Verbindungen, die der Formel IV entsprechen, aber anstelle eines oder mehrerer H-Atome eine oder mehrere solvolysierbare Gruppe(n) enthalten, mit 3-Chinuclidinon. So können insbesondere Verbindungen der Formel I, die am aromatischen Ring eine Acyloxygruppe tragen, vorzugsweise eine Alkanoyl-, Alkylsulfonyl- oder Arylsulfonylgruppe mit jeweils bis zu 10 C-Atomen, wie z.B. Methan-, Benzol- oder p-Toluolsulfonyl, zu den entsprechenden unsubstituierten Hydroxyderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natriumoder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Verbindungen der Formel I, die eine O-Alkylgruppe enthalten, können durch Etherspaltung in die entsprechenden Hydroxyderivate überführt werden. Z.B. kann man die Ether spalten durch Behandeln mit Dimethylsulfid-Bortribromid- Komplex, z.B. in Toluol, Ethern wie THF oder Dimethylsulf- oxid, oder durch Verschmelzen mit Pyridin- oder Anilin-hydrohalogeniden, vorzugsweise Pyridinhydrochlorid, bei etwa 150-250°.

Die Verbindungen der Formel I können unter Umständen ein oder mehrere Asymmetriezentren besitzen, sofern einer der Reste R¹-R⁵ chiral ist. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure , Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure,Isonicotinsäure, Methanoder Ethansulfonsäure, Ethandisulfonsäure, 2-Hydroxyethansulfonsäure, Benzolsulfonsäure, p-Toluolsulfonsäure, Naphthalinmono- und -disulfonsäuren, Laurylschwefelsäure.

In jenen Fällen, wo die Verbindungen der Formel I über acide phenolische OH-Gruppen verfügen, kann durch Behandlung mit Basen eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für parenterale oder topische Applikation eignen und mit den neuen Verbindungen nicht reagieren, beispielsweise Wasser, pflanzliche Öle, Benzylalkohole, Polyethylenglykole, Gelatine, Talk, Vaseline. Zur parenteralen Applikation eignen sich Lösungen, vorzugsweise ölige oder wässerige Lösungen, ferner Suspensionen, Emulsionen oder Implantate, für die topische Anwendung Salben, Cremes, Gels, Sticks oder Puder.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung und zur Prävention von dermatologischen Erkrankungen des menschlichen oder tierischen Körpers verwendet werden. Sie eignen sich zur Behandlung von zahlreichen Erkrankungen der Haut, insbesondere solchen, die durch intensive Sonneneinstrahlung hervorgerufen wurden. Die Verbindungen der Formel I zeigen eine signifikante pharmakologische Aktivität auf dem Gebiet der präventiven Behandlung von Entzündungen und/oder Hautallergien.

Dabei werden die erfindungsgemäßen Substanzen in der Regel in Analogie zu bekannten, im Handel befindlichen Präparaten verabreicht, parenteral, vorzugsweise in Dosierungen zwischen etwa 0,1 und 400 mg, insbesondere zwischen 0,2 und 50 mg pro Dosierungseinheit. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, Arzneistoffkombination und Schwere der jeweiligen Erkrankung, welcher die Therapie gilt. Die topische Applikation ist bevorzugt.

Ein weiterer Gegenstand der Erfindung ist die Verwendung der Verbindungen der Formel I zur Herstellung von kosmetischen Zubereitungen, welche in kosmetisch verträglichen Trägersubstanzen eine wirksame Menge mindestens eines Benzyliden-Chinuclidinon-Derivates der Formel I enthalten.

Insbesondere bevorzugt sind solche kosmetischen Zubereitungen, worin der Träger mindestens eine Fettphase aufweist, bzw. solche, worin der Träger mindestens eine wäßrige Phase aufweist.

Das erfindungsgemäße kosmetische Mittel kann als Mittel zum Schutz der menschlichen Epidermis und/oder der Haare oder als Sonnenschutzmittel verwendet werden.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch, in Form ölig-alkoholischer, öligwäßriger oder wäßrig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 1 bis 10 % bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglycol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gels enthalten außerdem natürliches oder synthetisches Öl oder Wachs.

Die festen Stifte enthalten natürliche oder synthetische Wachse und Öle, Fettalkohole, Fettsäureester, Lanolin und andere Fettkörper.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shamponieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel ebenfalls 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I in Kombination mit geeigneten UV-B-Filtern als kosmetische Produkte.

Als UV-B-Filter können handelsübliche Produkte, wie z.B. Eusolex® 6300 oder Eusolex® 232 verwendet werden.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natrium- oder Magnesiumsulfat, filtriert, dampft ein und reinigt durch Chromatographie an Kieselgel und/oder durch Kristallisation. Sämtliche Temperaturen sind in °C angegeben. Die UV-Messungen wurden nach herkömmlichen Methoden durchgeführt, wobei 1 mg der jeweiligen Substanz in 100 ml Ethanol gelöst wurde (c = 1 mg/100 ml). Aus dem meßtechnisch ermittelten Wert λₘₐₓ wurde die jeweilige Extinktion rechnerisch ermittelt.

### Beispiel 1

Eine Lösung von 16,0 g 3-Chinuclidinon (Hydrochlorid) in 100 ml Wasser wird mit 100 ml einer 32 %igen Natronlauge versetzt. Anschließend fügt man unter Rühren 36,0 g 4-(2-Ethyl-hexyloxy)-benzaldehyd [herstellbar aus p-Hydroxybenzaldehyd durch Umsetzung mit 1-Chlor-2-ethylhexan], gelöst in 250 ml Ethanol, hinzu und erhitzt 3 Std. unter Rückfluß. Nachdem die Reaktion abgeschlossen ist, wird wie üblich aufgearbeitet. Man erhält 2-[4-(2-Ethylhexyloxy)-benzyliden]-chinuclidin-3-on in Form eines hellgelben Feststoffes; F. 37°, λ max = 331 nm, E = 0,8.

### Analog erhält man durch Umsetzung von 3-Chinuclidinon (Hydrochlorid)

mit 4-(2-Ethyl-hexyloxy)-3-methoxy-benzaldehyd
   2-[4- (2-Ethyl-hexyloxy] -3-methoxy-benzyliden] -chinuclidin-3-on, F. 58°, λ max = 342 nm, E = 0,6;
mit 3-(2-Ethyl-hexyloxy)-4-methoxy-benzaldehyd
   2-[3-(2-Ethyl-hexyloxy)-4-methoxy-benzyliden]-chinuclidin-3-on, ölartig, λ max = 341 nm, E = 0,56;
mit 4-Methoxy-benzaldehyd
   2-(4-Methoxy-benzyliden)-chinuclidin-3-on, F. 123°, λ max = 327 nm, E = 1,1;
mit 3,4-Dimethoxy-benzaldehyd
   2- (3,4-Dimethoxy-benzyliden)-chinuclidin-3-on,
   F. 103°, λ max. = 338 nm, E = 0,8;
mit 2,4,5-Trimethoxy-benzaldehyd
   2- (2,4,5-Trimethoxy-benzyliden) -chinuclidin-3-on,
   F. 119°, λ max = 370 nm, E = 0,6.

### Beispiel 2

### Analog Beispiel 1 erhält man durch Umsetzung von 3-Chinuclidinon

mit 4-(2-Ethyl-pentyloxy)-2-methoxy-benzaldehyd
   2-[4-(2-Ethyl-pentyloxy)-2-methoxy-benzyliden]-chinuclidin-3-on,
mit 4- (2-Methyl-propyloxy)-3-methoxy-benzaldehyd
   2-[4-(2-Methyl-propyloxy)-3-methoxy-benzyliden]-chinuclidin-3-on,
mit 3-Ethoxy-4-methoxy-benzaldehyd
   2-(3-Ethoxy-4-methoxy-benzyliden)-chinuclidin-3-on,
mit 4-Propoxy-benzaldehyd
   2-(4-Propoxy-benzyliden)-chinuclidin-3-on,
mit 2,4-Dimethoxy-benzaldehyd
   2-(2,4-Dimethoxy-benzyliden)-chinuclidin-3-on,
mit 2,4,6-Trimethoxy-benzaldehyd
   2-(2,4,6-Trimethoxy-benzyliden)-chinuclidin-3-on,
mit 4-(2-Ethyl-hexyloxy)-3-methyl-benzaldehyd
   2-[4-(2-Ethyl-hexyloxy)-3-methyl-benzyliden]-chinuclidin-3-on,
mit 4-(2-Ethyl-hexyloxy)-3-hydroxy-benzaldehyd
   2-[4-(2-Ethyl-hexyloxy]-3-hydroxy-benzyliden]-chinuclidin-3-on,
mit 4-Ethoxy-benzaldehyd
   2-(4-Ethoxy-benzyliden)-chinuclidin-3-on,
mit3,4-Diethoxy-benzaldehyd
   2-(3,4-Diethoxy-benzyliden)-chinuclidin-3-on,
mit 2,6-Dimethyl-4-methoxy-benzaldehyd
   2-(2,6-Dimethyl-4-methoxy-benzyliden)-chinuclidin-3-on,
mit 4-Tert.-butyloxy-benzaldehyd
   2-(4-Tert.-butyloxy-benzyliden)-chinuclidin-3-on,
mit 3-(2-Methyl-butyloxy)-4-methoxy-benzaldehyd
   2-[3-(2-Methyl-butyloxy)-4-methoxy-benzyliden]-chinuclidin-3-on,
mit 2-Methyl-4-methoxy-benzaldehyd
   2-(2-Methyl-4-methoxy-benzyliden)-chinuclidin-3-on,
mit 2,5-Dihydroxy-4-methoxy-benzaldehyd
   2-(2,5-Dihydroxy-4-methoxy-benzyliden)-chinuclidin-3-on,
mit 2,6-Dihydroxy-4-methoxy-benzaldehyd
   2-(2,6-Dihydroxy-4-methoxy-benzyliden)-chinuclidin-3-on,
mit 2,6-Diethyl-4-methoxy-benzaldehyd
   2-(2,6-Diethyl-4-methoxy-benzyliden)-chinuclidin-3-on,
mit 2,6-Di-tert.-butyl-4-methoxy-benzaldehyd
   2-(2,6-Di-tert.-butyl-4-methoxy-benzyliden)-chinuclidin-3-on,
mit 2,6-Diisopropyl-4-methoxy-benzaldehyd
   2-(2,6-Diisopropyl-4-methoxy-benzyliden)-chinuclidin-3-on,
mit 3,5-Dihydroxy-4-methoxy-benzaldehyd
   2-(3,5-Dihydroxy-4-methoxy-benzyliden)-chinuclidin-3-on,
mit 3,5-Dimethyl-4-methoxy-benzaldehyd
   2-(3,5-Dimethyl-4-methoxy-benzyliden)-chinuclidin-3-on,
mit 3,5-Diisopropyl-4-methoxy-benzaldehyd
   2-(3,5-Diisopropyl-4-methoxy-benzyliden)-chinuclidin-3-on.

In den folgenden Anwendungsbeispielen A bis D und H können wahlweise jeweils folgende UV-Filter eingesetzt werden:
- 1.: 10 % Verbindung aus Beispiel 1
   - 2.: 5 % Verbindung aus Beispiel 1
   2 % Benzophenon-3
   3 % Octyldimethyl-p-aminobenzoesäureester (Octyldimethylpaba)
   - 3.: 6 % Verbindung aus Beispiel 1
   3 % 3-(4-Methylbenzyliden)-campher
   - 4.: 4 % Verbindung aus Beispiel 1
   1 % Octyltriazon
   - 5.: 8 % Verbindung aus Beispiel 1
   6 % Octylmethoxycinnamat
   - 6.: 6 % Verbindung aus Beispiel 1
   4 % Octyl-Salicylat
   2 % 4-Isopropyldibenzoylmethan
   - 7.: 8 % Verbindung aus Beispiel 1
   2 % Benzophenon-3

Die nachfolgend in Klammern angegebenen Firmenbezeichnungen geben die Bezugsquelle der jeweiligen Ingredienzien an.

### Anwendungsbeispiele

### Beispiel A: Sonnenschutzmittel (O/W)

Man mischt folgende Ingredienzien, wobei sie zum Homogenisieren gegebenenfalls auf 70-85 °C erhitzt werden; das Parfumöl wird bei 35-45 °C zugegeben.

| | Gew. % |
|---|---|
| UV-Filter | q.s. |
| | |
| Arlacel 165® "Glycerinmonostearat und POÄ-"Stearat" (ICI, Essen) | 9,5 |
| | |
| Atlas G-1790® "Polyoxyethylen(20)lanolin-Derivat" (ICI, Essen) | 6,60 |
| | |
| Lanette O® "Cetylstearylalkohol", (Henkel AG) | 3,00 |
| | |
| Paraffinöl dünnflüssig (E. Merck) | 3,00 |
| | |
| Isopropylmyristat | 1,50 |
| | |
| Abil AV 20® | 1,00 |
| | |
| Antioxidans enthaltend Butylhydroxytoluol "Oxynex 2004®" (E. Merck) | 0,02 |
| | |
| Allantoin (E. Merck) | 0,30 |
| | |
| Sorbit "Karion® F flüssig" (E. Merck) | 6,00 |
| | |
| Dinatriumsalz der Ethylendiamintetraessigsäure "Titriplex®" (E. Merck) | 0,05 |
| | |
| Pantothenylalkohol | 0,30 |
| | |
| Konservierungsmittel (E. Merck) | 0,5 |
| | |
| demineralisiertes Wasser | ad 100 |

### Beispiel B: Sonnenschutzmilch (O/W)

| | Gew. % |
|---|---|
| UV-Filter | q.s. |
| | |
| Mischung aus Cetyl-Dimethicone Copolyol, Polyglyceryl-4-Isostearat und Hexyllaurat "Abil WE 09®" (Th. Goldschmidt) | 5,00 |
| | |
| Paraffinöl dünnflüssig (E. Merck) | 16,00 |
| | |
| Natriumchlorid (E. Merck) | 2,00 |
| | |
| Glycerin (E. Merck) | 3,00 |
| | |
| Konservierungsmittel (E. Merck) | q.s. |
| | |
| Wasser, demineralisiert | ad 100,00 |
| | |
| Parfümöl Delaila® (Dragoco) | 0,50 |

Man mischt die Ingredienzien, wobei sie zum Homogenisieren gegebenenfalls auf 70-85 °C erhitzt werden; das Parfümöl wird bei 35-45 °C zugegeben.

### Beispiel C: Sonnenschutzcreme (W/O)

| | | Gew. % |
|---|---|---|
| A | UV-Filter | q.s. |
| | | |
| | POE Glycerol sorbitan oleostearat "Arlacel 581®" (ICI) | 7,0 |
| | | |
| | Paraffinöl dünnflüssig (E. Merck) | 6,0 |
| | | |
| | PPG-15 Stearylether und Cyclomethicon 10 "Arlamol S 7®" (ICI) | 2,0 |
| | | |
| | 020 Kevit-Wachs "Lunacera M®" (LW Fuller) | 5,0 |
| | | |
| | Cyclomethicon "Dow Corning 344®" (Dow Corning) | 4,00 |
| | | |
| | Triglyceridöl "Miglyol 812®" (Hülls Troisdorf AG) | 2,00 |
| | | |
| B | Glycerin (Art.-Nr. 4093) (E. Merck) | 2,00 |
| | | |
| | Magnesiumsulfat-Heptahydrat (E. Merck) | 0,17 |
| | | |
| | Konservierungsmittel (E. Merck) | q.s. |
| | | |
| | Wasser, demineralisiert | ad 100,00 |

Die Ingredienzien werden bei 70-85 °C homogenisiert; die Zubereitung wird gegebenenfalls bei 35-45 °C parfümiert.

### Beispiel D: Sonnenschutzcreme (O/W)

| | | | Gew. % |
|---|---|---|---|
| A | UV-Filter | | q.s. |
| | | | |
| | Mischung aus Cetearylalkohol und Ceteareth-20 "Emulgade 1000 Ni®" (Henkel) | (2) | 10,00 |
| | | | |
| | Paraffinöl dickflüssig (E. Merck) | (1) | 2,00 |
| | | | |
| | Dimethylsiloxan "Dow Corning 200 (100 cs)®" | (3) | 0,50 |
| | | | |
| | Oxynex 2004 (E. Merck) | (1) | 0,10 |
| | | | |
| B | Glycerin (E. Merck) | (1) | 5,00 |
| | | | |
| | Titriplex III® (E. Merck) | (1) | 0,10 |
| | | | |
| | Konservierungsmittel (E. Merck) | (1) | q.s. |
| | | | |
| | Wasser, demineralisiert | | ad 100,00 |

Die Ingredienzien werden bei 70 bis 85 °C homogenisiert. Die Zubereitung wird gegebenenfalls bei 35 bis 45 °C parfümiert.

### Beispiel E: Sonnenschutzcreme (O/W)

| | | Gew. % |
|---|---|---|
| Verbindung aus Beispiel 1 | | 5,00 |
| | | |
| Stearinsäure (E. Merck) | | 20,00 |
| | | |
| Triglycerid-Öl "Miglyol 812®" (Hüls Troisdorf AG) | | 20,00 |
| | | |
| UV-Filter 2-Phenylbenzimidazol-5-sulfonsäure "Eusolex 232®" (E. Merck) | | 1,50 |
| | | |
| Tris-(hydroxymethyl)-aminomethan (E. Merck) | | 0,66 |
| | | |
| Sorbit "Karion F flüssig®" (E. Merck) | | 5,00 |
| | | |
| Allantoin (E. Merck) | (1) | 0,20 |
| | | |
| Triethanolamin (E. Merck) | (1) | 6,00 |
| | | |
| Konservierungsmittel (E. Merck) | (1) | q.s. |
| | | |
| Wasser, demineralisiert | | ad 100,00 |

Zur Neutralisierung von Eusolex® 232 wird das Tris-(hydroxymethyl)-aminomethan in Wasser gelöst und Eusolex® 232 unter Rühren zugegeben. Nach vollständiger Lösung werden die restlichen Ingredienzien bei 75-85 °C hinzugefügt und homogenisiert. Unter Rühren abkühlen und gegebenenfalls bei 40 °C parfümieren.

### Beispiel F: Frisiergel (ölhaltig) mit UV-Filter

Die Verbindung aus Beispiel 1 kann auch durch ein Gemisch aus 2 % Verbindung aus Beispiel 1 und 1 % Benzophenon-3 bzw. 1 % 3-(4-Methylbenzyliden)-campher ersetzt werden.

| | | Gew. % |
|---|---|---|
| Verbindung aus Beispiel 1 | | 3 |
| | | |
| Polyoxyethylen-30-cetylstearylalkohol "Emulgin B 3®" (Henkel AG) | (2) | 13,00 |
| | | |
| Polyolfettsäureester "Cetiol HE®" (Henkel AG) | (2) | 20,00 |
| | | |
| Eutanol G® "Octyldecanol" (Henkel AG) | (2) | 5,00 |
| | | |
| Konservierungsmittel (E. Merck) | | q.s. |
| | | |
| Wasser, demineralisiert | | ad 100,00 |

Die Ingredienzien werden gemischt und bei 70 bis 85 °C homogenisiert. Die Zubereitung wird gegebenenfalls bei 35 bis 45 °C parfümiert.

### Beispiel G: Haarkur mit UV-Filter (Creme O/W)

| | Gew. % |
|---|---|
| UV-Filter | 3,00 |
| | |
| Cetylstearylalkohol "Lanette O®" (Henkel AG) | 2,50 |
| | |
| Polyoxyethylen-20-stearylalkohol "Emulgin B 2®" (Henkel AG) | 1,00 |
| | |
| Wachsester (Jojobaöl-Substitut) "Cetiol J 600®" (Henkel AG) | 1,00 |
| | |
| N-Cetyl-N,N,N-trimethylammoniumbromid (E. Merck) | 1,00 |
| | |
| Konservierungsmittel (E. Merck) | q.s. |
| | |
| Wasser, demineralisiert | ad 100,00 |

Die Herstellung erfolgt analog Beispiel A.

### Beispiel H: Sonnenöl

| | | Gew. % |
|---|---|---|
| UV-Filter | | q.s. |
| | | |
| POE-40-Sorbitolheptaoleat "Arlatone T®" (ICI) | | 2,00 |
| | | |
| Triglycerid-Öl "Miglyol 812®" (Hüls Troisdorf AG) | (3) | 16,00 |
| | | |
| Di-n-butyladipat "Cetiol B®" (Henkel AG) | (4) | 22,50 |
| | | |
| Isopropylmyristat (Henkel AG) | (4) | 7,50 |
| | | |
| Paraffinöl dünnflüssig (E. Merck) | | ad 100,00 |
| | | |
| Antioxidans enthaltend Butylhydroxytoluol "Oxynex 2004®" (E. Merck) | | 0,2 |
| | | |
| Konservierungsmittel | | q.s. |
| | | |
| Parfümöl 72979 (Haarmann & Reimer) | | q.s. |

Die Herstellung erfolgt analog Beispiel A.

### Beispiel I: Sonnenschutzgel (wässrig-alkoholisch)

### Wässrige Phase:

| | Gew. % |
|---|---|
| Verbindung aus Beispiel 2 | 10,00 |
| | |
| Tris-(hydroxymethyl)-aminomethan (E. Merck) | 2,80 |
| | |
| Allantoin (E. Merck) | 0,20 |
| | |
| Sorbit "Karion F® flüssig" (E. Merck) | 5,00 |
| | |
| Konservierungsmittel | q.s. |
| | |
| Wasser, demineralisiert | ad 100,00 |

### Alkoholische Phase:

| | Gew. % |
|---|---|
| Parfümöl (Haarmann & Reimer) | 0,30 |
| | |
| POE-35 hydriertes Rizinusöl "Arlatone 980®" (ICI) | 1,00 |
| | |
| Carboxyvinyl-Polymeres "Carbopol 940®" (Goodrich) | 1,50 |
| | |
| Wasser, demineralisiert | 35.50 |
| | |
| Triethanolamin (E. Merck) | 3,00 |
| | |
| Ethanol (96%ig) (E. Merck) | ad 100,00 |

Das Tris-(hydroxymethyl)-aminomethan wird in Wasser gelöst und das UV-Filter aus Beispiel 2 unter Rühren zugegeben. Nach voll- ständiger Lösung werden die restlichen Rohstoffe zugefügt und auf 75 °C erhitzt. Anschließend wird unter Rühren abgekühlt.

### Beispiel J: Sonnenschutzcreme (O/W)

| | | | % |
|---|---|---|---|
| A | UV-Filter | | q.s. |
| | | | |
| | Stearinsäure (E. Merck) | | 20,00 |
| | | | |
| | Triglycerid-Öl "Miglyol 812" | | 20,00 |
| | | | |
| B | Verbindung aus Beispiel 2 (E. Merck) | (1) | 10,00 |
| | | | |
| | Tris- (hydroxymethyl) -aminomethan (E. Merck) | (1) | 2,80 |
| | | | |
| | Sorbit "Karion F® flüssig" (E. Merck) | (1) | 5,00 |
| | | | |
| | Allantoin (E. Merck) | (1) | 0,20 |
| | | | |
| | Triethanolamin (E. Merck) | (1) | 6,00 |
| | | | |
| | Konservierungsmittel (E. Merck) | (1) | q.s. |
| | | | |
| | Wasser, demineralisiert | | ad 100,00 |

Als UV-Filter können wahlweise die Verbindungen aus Beispiel 1 oder 2 eingesetzt werden.

### Herstellung:

Das Tris-(hydroxymethyl)-aminomethan wird im Wasser der Phase B gelöst und das UV-Filter aus Beispiel 4 unter Rühren zugegeben. Nach vollständiger Lösung werden die restlichen Rohstoffe der Phase B zugegeben, und es wird auf 80° erhitzt. Die Phase A wird auf 75° erhitzt. Die Phase B wird langsam in Phase A eingerührt, homogenisiert, unter Rühren abgekühlt und gegebenenfalls bei 40° parfümiert.

### Beispiel K: Sonnenschutzcreme (O/W)

| | | | % |
|---|---|---|---|
| A | UV-Filter | | q.s. |
| | | | |
| | Stearinsäure (E. Merck) | | 20,00 |
| | | | |
| | Triglycerid-Öl "Miglyol 812®" (Hüls Troisdorf) | | 20,00 |
| | | | |
| B | Verbindung aus Beispiel 2 (E. Merck) | (1) | 5,00 |
| | | | |
| | Natriumhydroxid (10%ige Lösung) (E. Merck) | (1) | 0,40 |
| | | | |
| | Sorbit "Karion F® flüssig" (E. Merck) | (1) | 5,00 |
| | | | |
| | Allantoin (E. Merck) | (1) | 0,20 |
| | | | |
| | Triethanolamin (E. Merck) | (1) | 6,00 |
| | | | |
| | Konservierungsmittel | (1) | q.s. |
| | | | |
| | Wasser, demineralisiert | | ad 100,00 |

Als UV-Filter können wahlweise die Verbindungen aus Beispiel 1 oder 2 eingesetzt werden.

### Herstellung:

Der UV-Filter wird zur Neutralisierung in die Natriumhydroxidlösung und das Wasser der Phase B eingerührt. Nach vollständiger Lösung werden die restlichen Rohstoffe der Phase B zugegeben und auf 80° erhitzt. Die Phase A wird auf 75° erhitzt. Die Phase B wird langsam in Phase A eingerührt, homogenisiert, unter Rühren abgekühlt und gegebenenfalls bei 40° parfümiert.

### Beispiel L: Sonnenschutzcreme (O/W)

| | | | % |
|---|---|---|---|
| A | UV-Filter | | q.s. |
| | | | |
| | Stearinsäure (E. Merck) | (1) | 20,00 |
| | | | |
| | Triglycerid-Öl "Miglyol 812®" (Hüls Troisdorf AG) | (2) | 20,00 |
| | | | |
| B | UV-Filter aus Beispiel 2 (E. Merck) | (1) | 5,00 |
| | | | |
| | Sorbit "Karion F® flüssig" (E. Merck) | (1) | 5,00 |
| | | | |
| | Allantoin (E. Merck) | (1) | 0,20 |
| | | | |
| | Triethanolamin (E. Merck) | (1) | 6,17 |
| | | | |
| | Konservierungsmittel (E. Merck) | (1) | q.s. |
| | | | |
| | Wasser, demineralisiert | | ad 100,00 |

Als UV-Filter können wahlweise die Verbindungen aus Beispiel 1 oder 2 eingesetzt werden.

### Herstellung:

Das Triethanolamin wird im Wasser der Phase B gelöst und das UV-Filter aus Beispiel 4 unter Rühren zugegeben. Nach vollständiger Lösung werden die restlichen Rohstoffe der Phase B zugegeben und auf 80° erhitzt. Die Phase A wird auf 75° erhitzt. Die Phase B wird langsam in Phase A eingerührt, homogenisiert, unter Rühren abgekühlt und gegebenenfalls bei 40° parfümiert.

## Patentansprüche

1. Kosmetische Zubereitung, **gekennzeichnet durch** einen Gehalt an mindestens einer Verbindung der Formel I 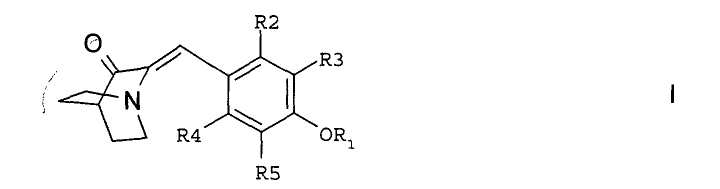 worin
R1 A
R2, R3, R4 und R5 jeweils unabhängig voneinander H, A, OA oder OH
und
A einen geradkettigen oder verzweigten Alkylrest mit
1 bis 10 C-Atomen bedeuten sowie ihre Salze.

2. Kosmetische Zubereitung nach Anspruch 1, **dadurch gekennzeichnet, daß** sie zusätzlich einen UV-B-Filter enthält.

3. Verwendung von Verbindungen der Formel I gemäß Anspruch 1 zur Herstellung von kosmetischen Produkten.

4. Verfahren zur Herstellung einer kosmetischen Zubereitung, **dadurch gekennzeichnet, daß** man eine Verbindung der Formel I gemäß Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Verfahren zur Herstellung einer pharmazeutischen Zubereitung, **dadurch gekennzeichnet, daß** man eine Verbindung der Formel I gemäß Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

6. Pharmazeutische Zubereitung, **gekennzeichnet durch** einen Gehalt an mindestens einer Verbindung der Formel I gemäß Anspruch 1 und/oder einem ihrer physiologisch unbedenklichen Salze.

7. Verwendung einer Verbindung der Formel I gemäß Anspruch 1 und/oder eines ihrer physiologisch unbedenklichen Salze zur Herstellung von Arzneimitteln.

8. Benzyliden-chinuclidinon-Derivate der Formel II 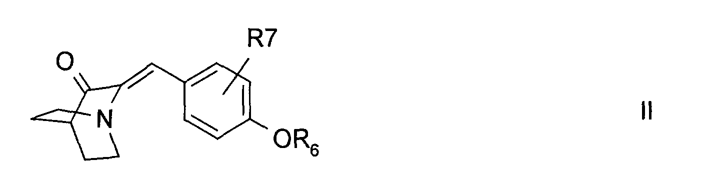 worin
R6 2-Ethylhexyl,
R⁷ H oder OA und
A C₁₋₄ Alkyl,
bedeuten, sowie ihre Salze.

9. a) 2-(4-(2-Ethylhexyloxy)-benzyliden)-chinuclidin-3-on;
b) 2-(4-(2-Ethylhexyloxy)-3-methoxy-benzyliden)-chinuclidin-3-on;

10. Verfahren zur Herstellung von Verbindungen der Formel II gemäß Anspruch 8, **dadurch gekennzeichnet, daß** man 3-Chinuclidinon mit einer Verbindung der Formel III 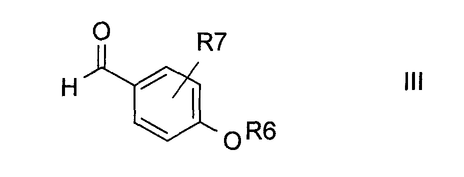 worin die Reste R¹ und R² die in Anspruch 8 angegebene Bedeutung haben,
umsetzt,
oder, daß man eine sonst der Formel II entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder, daß man eine erhaltene Base der Formel II durch Behandeln mit einer Säure in eines ihrer Salze umwandelt.

## Claims

1. Cosmetic preparation, **characterised in that** it contains at least one compound of the formula I 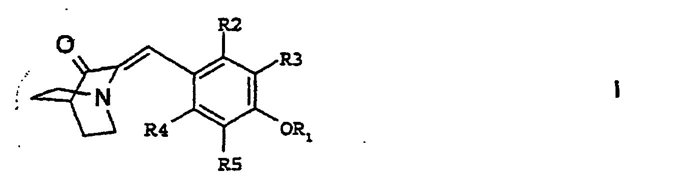 in which
R1 is A,
R2, R3 R4 and R5 are in each case independently of one another H, A, OA or OH
and
A is a straight-chain or branched alkyl radical having 1 to 10 C atoms, and their salts.

2. Cosmetic preparation according to Claim 1, **characterised in that** it additionally contains a UV-B filter.

3. Use of compounds of the formula I, according to Claim 1, for the production of cosmetic products.

4. Process for the production of a cosmetic preparation, **characterised in that** a compound of the formula I, according to Claim 1, and/or one of its physiological acceptable salts is brought into a suitable dosage form together with at least one solid, liquid or semi-liquid excipient or auxiliary.

5. Process for the production of a pharmaceutical preparation, **characterised in that** a compound of the formula I, according to Claim 1, and/or one of its physiological acceptable salts is brought into a suitable dosage form together with at least one solid, liquid or semi-liquid excipient or auxiliary.

6. Pharmaceutical preparation, **characterised in that** it contains at least one compound of the formula I, according to Claim 1, and/or one of its physiologically acceptable salts.

7. Use of a compound of the formula I, according to Claim 1, and/or one of its physiologically acceptable salts for the production of medicaments.

8. Benzylidenequinuclidinone derivatives of the formula II 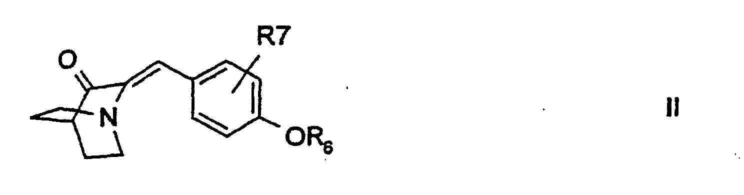 in which
R6 is 2-ethylhexyl,
R⁷ is H or OA, and
A is C₁₋₄alkyl,
and their salts.

9. a) 2- (4- (2-Ethylhexyloxy)benzylidene)quinuclidin-3-one;
b) 2- (4- (2-ethylhexyloxy) -3-methoxybenzylidene)-quinuclidin-3-one.

10. Process for preparing compounds of the formula II according to Claim 8, **characterised in that** 3-quinuclidinone is reacted with a compound of the formula III 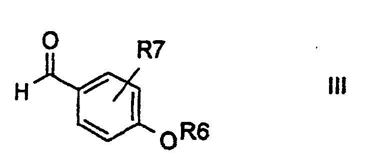 in which the radicals R¹ and R² have the meaning given in Claim 8,
or **in that** a compound which otherwise corresponds to the formula II but which instead of one or more hydrogen atoms contains one or more solvolysable groups is treated with a solvolyzing agent,
and/or **in that** a resulting base of the formula II is converted into one of its salts by treatment with an acid.

## Revendications

1. Préparation cosmétique, **caractérisée en ce qu'**elle contient au moins un composé de formule I 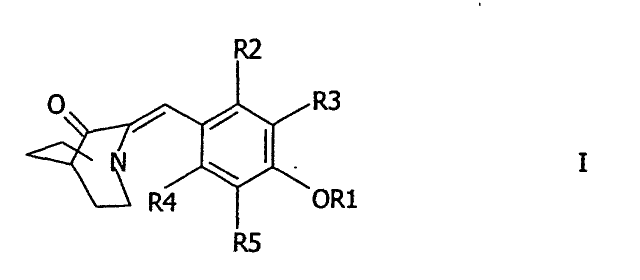 où
R¹ représente A,
R², R³, R⁴ et R⁵ représentent, indépendamment les uns des autres, H, A, OA ou OH et
A représente un reste alkyle linéaire ou ramifié comportant 1 à 10 atomes de C,
ainsi que ses sels.

2. Préparation cosmétique selon la revendication 1, **caractérisée en ce qu'**elle contient en plus un filtre U.V. B.

3. Utilisation des composés de formule I selon la revendication 1 pour la fabrication de produits cosmétiques.

4. Procédé pour la fabrication d'une préparation cosmétique, **caractérisé en ce que** l'on met sous une forme de dosage appropriée un composé de formule I selon la revendication 1 et/ou l'un de ses sels physiologiquement acceptables, associé à au moins un support ou un adjuvant solide, liquide ou semi-liquide.

5. Procédé pour la fabrication d'une préparation pharmaceutique, **caractérisé en ce que** l'on met sous une forme de dosage appropriée un composé de formule I selon la revendication 1 et/ou l'un de ses sels physiologiquement acceptables, associé à au moins un support ou un adjuvant solide, liquide ou semi-liquide.

6. Préparation pharmaceutique, **caractérisée en ce qu'**elle contient au moins un composé de formule I selon la revendication 1 et/ou l'un de ses sels physiologiquement acceptables.

7. Utilisation d'un composé de formule I selon la revendication 1 et/ou de l'un de ses sels physiologiquement acceptables pour la fabrication de médicaments.

8. Dérivés de la quinuclidinone benzylidène de formule II 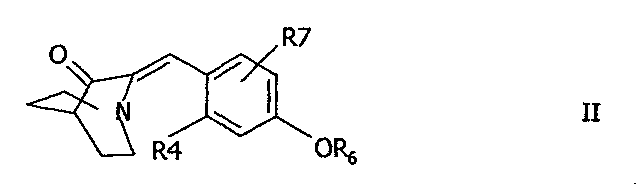 où
R⁶ représente le 2-éthylhexyle,
R⁷ H ou OA et
A un alkyle en C₁₋₄,
ainsi que leurs sels.

9. a) La 2-(4-(2-éthylhexyloxy)benzylidène)quinuclidin-3-one,
b) La 2-(4-(2-éthylhexyloxy)-3-méthoxybenzylidène)quinuclidin-3-one.

10. Procédé pour la préparation des composés de formule II selon la revendication 8, **caractérisé en ce que** l'on fait réagir la 3-quinuclidinone avec un composé de formule III 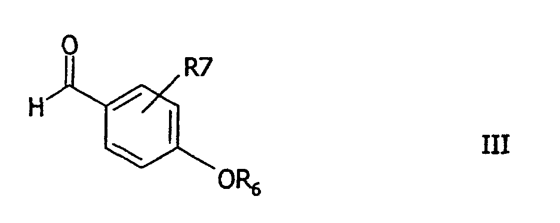 où les restes R¹ et R² ont les significations indiquées dans la revendication 8, ou **en ce que** l'on traite un composé correspondant à la formule II, mais qui contient à la place d'un ou plusieurs atomes d'hydrogène, un ou plusieurs groupes solvolysables, avec un agent solvolysant,
et/ou **en ce que** l'on transforme une base de formule II obtenue en l'un de ses sels, par traitement avec un acide.
